# EUROPEAN PATENT APPLICATION

(11) **EP 1 038 531 A2**
(43) Date of publication of application: **27.09.2000**
(21) Application number: 00105660.5
(22) Date of filing: 17.03.2000
(51) Int. Cl.: A61K 35/78, A23L 1/30

(54) **A composition comprising soybean isoflavones and a method for the production thereof**

(30) Priority: 18.03.1999 JP 7285599
(71) Applicant: Ajinomoto Co., Inc., Tokyo 104-8315 (JP)
(72) Inventor: Ozawa, Yoichi, c/o Ajinimoto Co., Inc., Kawasaki-shi, Kanagawa 210-8681 (JP); Kouda, Yuki, c/o Asahiyushi, Co., Asahikawa-shi, Hokkaido 078-8253 (JP); Kobayashi, Kenichi, c/o Asahiyushi, Co., Asahikawa-shi, Hokkaido 078-8253 (JP); Morinaga, Yasushi c/o Ajinomoto Co., Inc., Kawasaki-shi, Kanagawa 210-8681 (JP)
(74) Representative: Strehl Schübel-Hopf & Partner

(57) **Abstract**

The purpose of the present invention is to provide a soybean isoflavone mixture containing a high content of Genistein isoflavones which have physiologically advantageous effects, and a composition comprising said mixture.

That is, the present invention relates to a composition prepared from a germ-removed soybean, which comprises a soybean isoflavone mixture containing 50 % by weight or more, preferably 65 % by weight or more for Genistein isoflavones, and a method for the production of said composition.

## Description

### TECHNICAL FIELD

The invention relates to a composition prepared from a germ-removed soybean, which comprises a soybean isoflavone mixture, especially a soybean isoflavone mixture containing a high content of Genistein isoflavones, to a method for its production, and to a food comprising said composition.

### BACKGROUND ART

A soybean consists of a cotyledon (ca. 90%), a hull (ca. 6∼8%), and a germ (ca. 2∼4%), each part containing about 0.4%, 0.1% and 2%, respectively, of a series of compounds called "soybean isoflavones" (Kudou, S., et al., Agric. Biol. Chem., 55:9, 2227-2233 (1991)). The soybean isoflavones are generally classified into Daidzein, Genistein, and Glycitein groups.

Daidzein isoflavones include an aglycone named "Daidzein", its glycoside named "Daidzin", its malonic ester named "Malonyldaidzin" and a small amount of an acetylated glycoside named "Acetyldaidzin." Similarly, Genistein isoflavones include an aglycone named "Genistein", its glycoside named "Genistin", its malonic ester named "Malonylgenistin" and a small amount of an acetylated glycoside named "Acetylgenistin." And, Glycitein isoflavones include an aglycone named "Glycitein", its glycoside named "Glycitin", its malonic ester named "Malonylglycitin" and a small amount of an acetylated glycoside named "Acetylglycitin."

It is conceived that these glycosides and esters are hydrolyzed by a variety of enzymes of enteric bacteria such as glycosidase and esterase to give their corresponding aglycones which are then absorbed from the gut.

It was reported that the soybean isoflavones, especially the Genistein isoflavones had advantages to inhibit the occurrence of hormone-related cancers such as hysterocarcinoma, oophoroma, mastocarcinoma, prostatic carcinoma and the like, and hormone-independent cancers such as gastric carcinoma, and to inhibit adverse effects due to environmental hormones or endocrine disruptors (Surendra P. Verma and Barry R. Goldin; NUTRITION AND CANCER, 30 (3), 232 (1998)).

It was further reported that Genistein, as an agonist of estrogen, showed an activity of 1/1000 that of estrogen, and that it did not affect ovary, but did inhibit bone resorption so as to be effective in the prevention of osteoporosis (National Institute of Health and Nutrition, The 16^{th} Annual Meeting of The Japanese Society for Bone and Mineral Research, 1998.8.5∼6).

It was also reported that a mixture of 75% for Genistein and 25% for Daidzein significantly inhibited the occurrence of prostatic carcinoma in rats (University of Tsukuba, Medical branch, Urology, Japan Food & Research Laboratories, National Cancer Center-Japan, The 57^{th} Annual Meeting of the Japanese Cancer Association).

As seen from the above, the soybean isoflavones, especially Genistein, have physiologically advantageous activities for human body, and they are considered to be effective in the prevention of osteoporosis, cancers, disorders in circulatory organs, menopausal syndromes, etc.

As mentioned above, since the soybean germ has the highest content of the soybean isoflavones among the parts of a soybean, the soybean isoflavones have been conventionally produced using the germ as a starting material, by concentrating an extract of it with alcohol (such as hydrous ethanol and methanol), extracting the concentrate with water-butanol, and concentrating a butanol layer, optionally followed by fractionation by means of Sephadex (Kudou, S. et al., Agric. Biol. Chem., 55:3 859 (1991)).

The soybean isoflavones are similarly produced from defatted soybeans by the extraction with alcohol, followed by its concentration.

The soybean isoflavone mixture has been also produced from the waste water obtained during the soybean boiling process in a traditional fermented soybean paste production, using synthetic resin for the adsorption of isoflavones in the waste water followed by the elution with hydrous alcohol and the concentration of the alcoholic extract (Yoshimi Kitada, et al., Nippon Shokuhin Kogyo Gakkaishi, 33:12, 821 (1986)).

Furthermore, it has been produced from the broth obtained during an industrial production of cooked soybeans by adsorption using synthetic resin adsorbent and its elution with hydrous alcohol followed by the concentration of extract.

The adsorption of the soybean isoflavones with synthetic resins has been already carried out in the process of removing odors from the soybean protein extract (The Japanese Patent Publication Sho.62-25016).

However, the soybean isoflavone mixture prepared from the soybean germ by the above conventional method contains only about 10∼13 % by weight for Genistein isoflavones that are considered to be effective components of the isoflavones in the prevention of osteoporosis, cancers, disorders in circulatory organs, menopausal syndromes, etc. Further, the soybean isoflavone mixtures prepared from the defatted or whole soybean by the above conventional methods contain at most about 45 % by weight for Genistein isoflavones, and at most about 30∼40 % by weight for total soybean isoflavones (Table 1). A solid product prepared from the broth obtained during the production of the cooked soybeans by the above conventional method contains at most 28.2 % by weight for total soybean isoflavones .

**TABLE 1**

| Components' contents in the commercially available food products containing soybean isoflavones | | | | | | | |
|---|---|---|---|---|---|---|---|
| Material | Product | Manufacturer | Daidzein | Genistein | Glycitein | Total | (Content) |
| Soybean germ | FUJIFLAVONE | FUJICCO | ca.50% | ca.10% | ca.40% | 100 | |
| | FUJIFLAVONE P40 | | ca.20 | ca.4 | ca.16 | | 40% |
| | FUJIFLAVONE P10 | | ca.5 | ca.1 | ca.4 | | 10% |
| | FUJIFLAVONE C | | ca.1 | ca.0.2 | ca.0.8 | | 2% |
| | SoyLife | TRADEPIA | 9.88-11.66 mg/g | 2.59-3.13 mg/g | 7.30-10.38 mg/g | | |
| | | | 48 | 13 | 39 | 100 | |
| | | | ca.1.1% | ca.0.3% | ca.0.9% | | 2.0-2.5% |
| Soybean | SoyAct | Kikoman | 45 | 45 | 9 | 100 | |
| | | | 12-18% | 12-18% | 2-4% | | 30% or more |

### DISCLOSURE OF INVENTION

The present inventors have analyzed the amounts of soybean isoflavones contained in various soybean-related products in order to find out the most preferable source for the production of a soybean isoflavone mixture containing a lot of Genistein isoflavones that are considered to have physiologically advantageous effects. The results are shown in Table 2.

**TABLE 2**

| Amounts of the soybean isoflavones in the soybean-related products | | | | | | | | | |
|---|---|---|---|---|---|---|---|---|---|
| Soybean-related products | Whole soybean | Hull | Germ | Crude oil | Crude lecithin | Defatted soybean | Alcohol used for washing defatted soybean | Soy-cake | Whey |
| Contents of soybean isoflavones (%) | 0.39 | 0.10 | 1.56 | 0.00 | 0.05 | 0.41 | 0.11 | 0.003 | 0.01 |

As seen in Table 2, it has been now revealed that soybean whey, which is obtained in a large amount during the production process of soy protein from germ-removed, defatted soybeans and discarded as industrial waste water, also contains soybean isoflavones in a low concentration. As the soybean whey is an aqueous solution, it is conceived that the soybean isoflavones may be easily prepared from the soybean whey using a synthetic resin adsorbent.

The present inventors have actually found that the soybean isoflavone mixture prepared from the soybean whey consists mainly of Genistein and Daidzein isoflavones, but contains substantially no Glycitein isoflavones.

The present invention was made on the basis of the above findings. The present invention relates to a composition prepared from a germ-removed soybean, which comprises a soybean isoflavone mixture containing 50 % by weight or more, preferably 65 % by weight or more for Genistein isoflavones, and to a method for its production.

Desirably, the composition according to the present invention comprises a soybean isoflavone mixture containing 5 % by weight or less for, preferably 1 % by weight or less for, and most preferably substantially none for Glycitein isoflavones. Furthermore, the composition comprising 41 % by weight or more for the total soybean isoflavone mixture is preferred.

The kinds and amounts of other ingredients comprised in the present composition may vary depending on the production conditions such as the kinds of starting soybeans and an eluent, eluting conditions, etc.

### BRIEF DESCRIPTION OF THE DRAWINGS

Fig. 1 shows an elution pattern obtained by elution of the soybean isoflavones adsorbed on the synthetic resin adsorbent with hydrous ethanol (e.g., 60% ethanol).
Fig. 2 shows an HPLC chromatogram of the present composition comprising the soybean isoflavone mixture, which has been prepared using as a starting material the soybean whey that was obtained during the extraction of protein from the germ-removed and defatted soybeans.

### BEST MODE FOR CARRYING OUT THE INVENTION

The composition according to the present invention may be prepared, for example, using the soybean whey as a starting material, by adsorbing the isoflavones in the whey with the synthetic resin adsorbent, eluting and concentrating the resulting eluate.

The "germ-removed soybean" according to the present invention means a soybean from which at least 50%, preferably 85%, more preferably 90% of the germ part has been removed on the whole. It is preferred to remove the germ part at the highest ratio possible in order to reduce the content of Glycitein isoflavones.

The "soybean whey" means an aqueous layer that is obtained as a result of separation of protein by isoelectric precipitation from an aqueous solution of soybean protein prepared by extraction from soybean with water. The soybean whey may be prepared by a method well known to those skilled in the art.

In the production method according to the present invention, it is preferred to use the soybean whey as a starting material that is obtained during the extraction of protein from the germ-removed and defatted soybeans.

The soybean whey may be prepared, for example, firstly by flaking and defatting soybeans from which the germ and hull have been removed beforehand, secondly subjecting the resulting germ-removed and defatted soybeans to extraction with water, thirdly adjusting the resulting aqueous extract of soybean protein to ca. pH 4.5, and finally separating and collecting the precipitated protein.

Any adsorbent well known in the art may be used as the synthetic resin adsorbent in the present invention, one which can adsorb well a hydrophobic material with a molecular weight of 1,000 or less being preferred. For example, Sepabeads SP207 and Sepabeads SP900 (Mitsubishi Chemical Ltd.), and Diaion HP20 (Mitsubishi Chemical Ltd.) may be listed.

A well known eluent including hydrous alcohols such as 55-80% ethanol and methanol may be used to elute the soybean isoflavones adsorbed on the synthetic resin adsorbent.

Before adsorbing the isoflavones in the soybean whey onto the synthetic resin adsorbent, it can be adjusted to pH 7 so that the precipitation of the remaining protein in the soybean whey may be inhibited.

Other conditions in the present production method such as temperature, a ratio of the amount of the soybean whey to that of the synthetic resin adsorbent, and adjusting pH of the soybean whey may be optionally determined by those skilled in the art, depending on purpose and the like.

The present invention is explained more in detail by referring to the following examples, which should not be construed to limit the scope of the present invention.

The soybean isoflavones were analyzed under the following conditions.

### (Method for the analysis of the soybean isoflavones)

A sample solution (5 µl, 0.05mg/ml MeOH) was applied to the HPLC column (COSMOSIL Packed column 5C18-AR Waters: 250x4.6mm) kept at 35 °C, eluted with a linear gradient of 15-35% acetonitrile constantly containing 0.1 % acetic acid in 50 min at a flow rate of 1ml/min, and detected at 254 nm. Four commercially available compounds of Daidzein, Daidzin, Genistein,and Genistin were used as standard materials. On the other hand, Malonyldaidzin and Malonylgenistin, which were not commercially available, were identified in accordance with each peak in the HPLC results of S. Kudou, et al., Agric. Biol. Chem., 55(9), 2227-2233 (1991), and their molecular weights were corrected with the proviso that their absorbances were equal to those of Daidzin and Genistin, respectively, so that their concentrations in the sample could be estimated.

### EXAMPLE 1

The composition comprising a soybean isoflavone mixture according to the present invention was prepared from the soybean whey as a starting material that was obtained during the extraction process of soybean protein from the defatted soybeans, at least 90% of whose germ had been removed.

Thus, the synthetic resin adsorbent (100g; Sepabeads SP207) sufficiently activated by washing with methanol three times was then washed three times with distilled water. The soybean whey (100 l) containing about 0.01% by weight of the soybean isoflavones, which had been obtained during the extraction of soybean protein from the germ-removed and defatted soybeans, was applied to the resin adsorbent, and gently stirred overnight. Optionally, a supernatant may be discarded and the fresh soybean whey may be added and stirred further. The above procedures may be repeated several times until the soybean isoflavones are sufficiently adsorbed onto the resin.

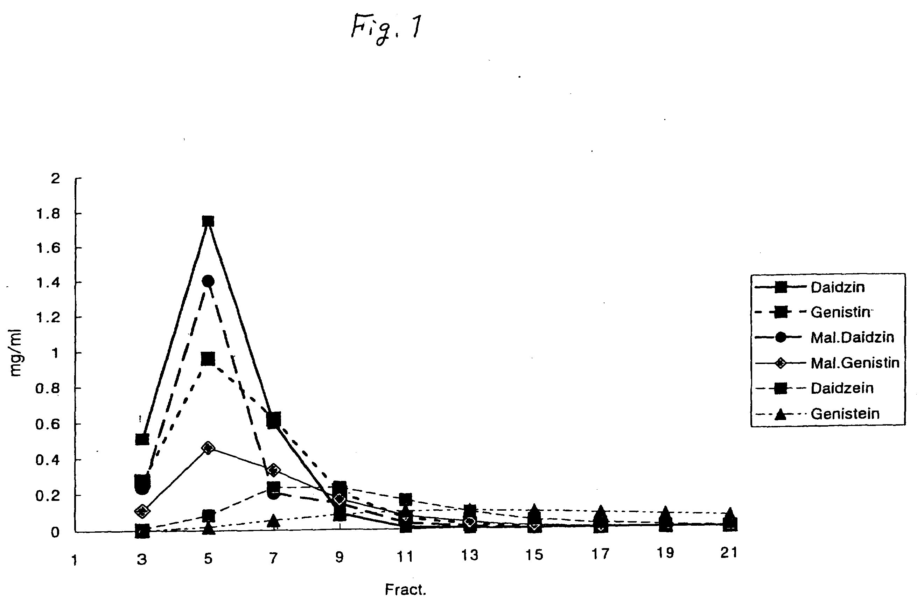

The synthetic resin adsorbent with the soybean isoflavones adsorbed thereon was collected and washed with water. Then it was packed into a column and the adsorbate was eluted with hydrous ethanol (e.g., 60% ethanol). The elution pattern is shown in Fig.1

The resulting brownish eluate was concentrated under a reduced pressure by means of an evaporator. The resulting concentrate was mixed with water to give slurry, which was then lyophilized. The lyophilized product was pulverized to obtain a brownish powder of the soybean isoflavone mixture (ca. 9.0g). The adsorption rate was therefore calculated to be about 9%.

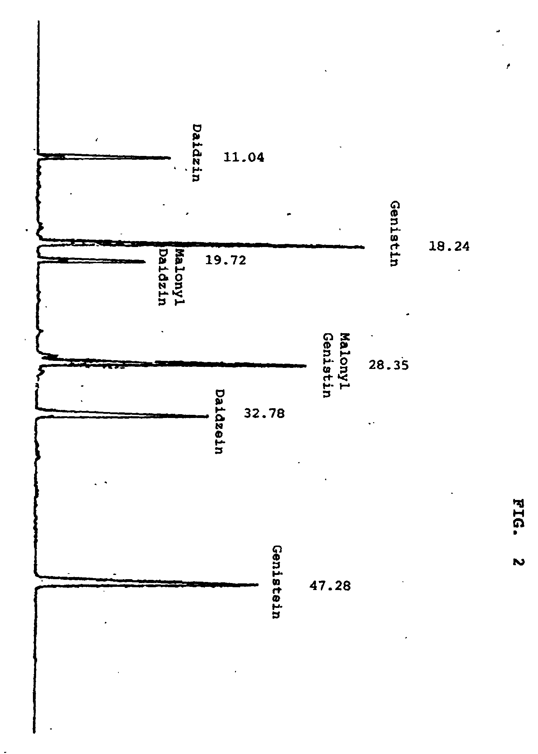

HPLC of the thus prepared composition comprising the soybean isoflavone mixture is shown in Fig. 2, and the analytical data are shown in Table 3.

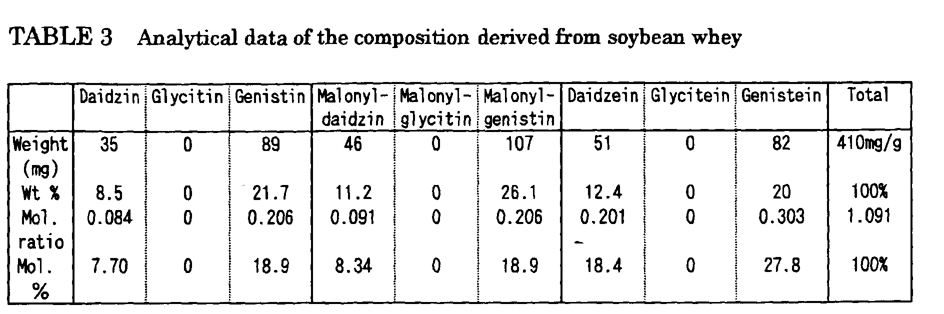

As seen from Table 3, 1g of the present composition comprises the isoflavone mixture of 410 mg in total (41% by weight). It has been revealed that the isoflavone mixture contains only Genistein and Daidzein isoflavones, and no Glycitein isoflavnes are detected.

Further, the ratio of Daidzein isoflavones and Genistein isoflavones in the above composition is about 34:66, as seen from Table 4.

**TABLE 4**

| Ratios of Daidzein, Genistein and Glycitein isoflavones in the composition derived from soybean whey | | | | |
|---|---|---|---|---|
| | Soybean isoflavone mixture | | | |
| | Daidzein | Glycitein | Genistein | Total |
| Mol.% | 34 | 0 | 66 | 100 |

Since the isoflavone mixture comprised in the present composition derived from the germ-removed soybeans consists mainly of Genistein and Daidzein isoflavones, these isoflavones may be easily purified into a mixture with a high purity using the composition according to the present invention as a starting material by means of recrystallization and so on.

Further, the present invention relates to a food comprising the above-presented composition. Thus, by making use of physiological activities of the isoflavones comprised in the present composition, the present composition may be used per se as nutritional fortified foods or foods for specified health use, or may be used as an ingredient (material) of those foods as well.

## Claims

1. A composition prepared from a germ-removed soybean, which comprises a soybean isoflavone mixture containing 50 % by weight or more of Genistein isoflavones.

2. The composition according to Claim 1 comprising a soybean isoflavone mixture containing 5 % by weight or less of Glycitein isoflavones.

3. The composition according to Claim 1 or 2 comprising 41 % by weight or more of a total soybean isoflavone mixture.

4. A method for the production of the composition according to any one of claims 1 to 3 using soybean whey as a starting material, which comprises the treatment of soy bean whey with a synthetic resin adsorbent to adsorb therefrom a soybean isoflavone mixture, eluting the isoflavones from the resin adsorbent and concentrating the eluate.
